# EUROPEAN PATENT APPLICATION

(11) **EP 4 501 411 A1**
(43) Date of publication of application: **05.02.2025**
(21) Application number: 22933655.7
(22) Date of filing: 13.12.2022
(51) Int. Cl.: A61Q 1/00, A61Q 19/00, A61K 8/02, A61K 8/31, A61K 8/34, A61K 8/73

(54) **OIL-BASED COSMETIC**

(30) Priority: 24.03.2022 JP 2022048647
(71) Applicant: Daio Paper Corporation, Ehime 799-0492 (JP)
(72) Inventor: SASAKI, Hiroto, Shikokuchuo-shi, Ehime 799-0492 (JP)
(74) Representative: Meissner Bolte Partnerschaft mbB
(86) International application number: PCT/JP2022/045762
(87) International publication number: WO 2023/181526

(57) **Abstract**

To provide an oil-based cosmetic that hardly imparts a foreign body feeling and suppresses stickiness and gloss.

This problem is solved by an oil-based cosmetic containing a fluid oil and a cellulose powder, in which the cellulose powder contains dried fine fibrous cellulose having an average fiber diameter of 1 to 1000 nm, has an average particle size of 1 to 100 µm, and has a specific surface area of 0.1 to 10 m²/g.

## Description

### Technical Field

The present invention relates to an oil-based cosmetic.

### Background Art

Conventionally, an oil-based cosmetic, for example, an external skin preparation such as petrolatum or a skin cosmetic such as a lip cream or a lipstick contains a high-viscosity hydrocarbon, and is therefore excellent in an adhesion feeling to the skin and sustainability due to a so-called emollient effect (moisturizing effect). On the other hand, these oil-based cosmetics are also likely to cause stickiness when applied to the skin. Various studies for reducing stickiness have been heretofore made, and examples thereof include mixing (emulsification) of petrolatum and a water-based moisturizing component, mixing of petrolatum and a liquid oil, and blending of an inorganic ultrafine particle powder into petrolatum.

Examples of a technique related to an oil-based cosmetic containing petrolatum include the following literatures. Patent Literature 1 proposes an oil-based cosmetic obtained by emulsifying petrolatum using polycondensed polymer particles or closed endoplasmic reticulum, and discloses that this oil-based cosmetic is excellent in a use feeling and can suppress viscosity and stickiness. Patent Literature 2 proposes that a powdery hydrophobically modified alkyl cellulose is blended in white petrolatum to reduce a sticky feeling. Patent Literature 3 proposes an oil-based cosmetic obtained by combining petrolatum with metal oxide ultrafine particles as an ultraviolet scattering agent and a volatile oil agent (volatile silicone oil), and discloses that the oil-based cosmetic has spreading performance and can suppress stickiness.

### Citation List

### Patent Literature

Patent Literature 1: WO 2019/021801 A
Patent Literature 2: JP 2014-141424 A
Patent Literature 3: JP 2015-229643 A
Patent Literature 4: JP 5-32519 A
Patent Literature 6: JP 2019-011287 A
Patent Literature 6: JP 2018-199671 A

### Summary of Invention

### Technical Problem

As described above, an object of Patent Literatures 1 to 3 is to suppress stickiness. However, on the other hand, when the closed endoplasmic reticulum of Patent Literature 1, the powdery hydrophobically modified alkyl cellulose of Patent Literature 2, or the metal oxide ultrafine particles of Patent Literature 3 are contained in an oil-based cosmetic, a foreign body feeling may be felt when the oil-based cosmetic is applied to the skin. In addition, in a case of an oil-based cosmetic, the inventors of the present invention consider that the degree of gloss is also an important property in addition to stickiness, but the degree of gloss is not considered in the above-described Patent Literatures.

Therefore, the present invention has been made in view of the above circumstances, and an object of the present invention is to provide an oil-based cosmetic that hardly imparts a foreign body feeling and suppresses stickiness and gloss.

### Solution to Problem

The problem is solved by the following aspects.

### (First Aspect)

An oil-based cosmetic including a fluid oil and a cellulose powder,
wherein the cellulose powder contains dried fine fibrous cellulose having an average fiber diameter of 1 to 1000 nm, has an average particle size of 1 to 100 µm, and has a specific surface area of 0.1 to 10 m²/g.

Patent Literature 1 reduces stickiness by adding polycondensed polymer particles or closed endoplasmic reticulum as an emulsifier. Similarly, Patent Literature 2 reduces stickiness by adding a hydrophobically modified alkyl cellulose, and Patent Literature 3 reduces stickiness by adding a volatile oil agent and hydrophobized metal oxide ultrafine particles. On the other hand, the present aspect reduces stickiness by inclusion of a fluid oil and a cellulose powder in an oil-based cosmetic. When the cellulose powder itself is added to a medium, a viscosity of the medium increases. Although this mechanism is not strictly clarified, it is presumed that this is probably due to an influence of a hydrogen bond formed by a hydroxy group and a hydrogen group included in cellulose. It is considered that molecules of cellulose are hydrogen-bonded to each other to construct a static and three-dimensional network structure, thereby resisting shear stress. It is known from measurement by the inventors that a cellulose powder has thixotropy, and has a relatively high viscosity in a stationary state and a low viscosity in a moving state. The oil-based cosmetic of the present embodiment contains the cellulose powder, and therefore has a high B-type viscosity at a low rotation speed, but decreases the B-type viscosity as the rotation speed is increased. As described above, an oil-based cosmetic having thixotropy decreases a viscosity and improves spreading performance due to application of an external force by a hand or the like at the time of application to the skin. Therefore, a user can apply the oil-based cosmetic while freely spreading the oil-based cosmetic.

The cellulose powder of the present embodiment contains dried fine fibrous cellulose processed from natural wood or the like as a raw material. A surface of the dried fine fibrous cellulose lacks smoothness, has irregularities, and is stiff when viewed microscopically, and thus has no gloss. Therefore, the oil-based cosmetic of the present aspect contains the cellulose powder, and therefore suppresses gloss of a portion where the oil-based cosmetic is applied.

The cellulose powder of the present aspect contains dried fine fibrous cellulose having an average fiber diameter of 1 to 1000 nm, has an average particle size of 1 to 100 µm, and has a specific surface area of 0.5 to 10 m²/g. The cellulose powder of the present aspect has a relatively large average particle size but has softness of cellulose fibers. Therefore, when the oil-based cosmetic of the present aspect is applied to the skin, a gritty feeling (foreign body feeling) is hardly felt.

The present inventors have found that a fluid oil imparts a stickiness feeling when applied to the skin, but stickiness is reduced when a cellulose powder is contained together with the fluid oil. This is probably due to thixotropy of the cellulose powder. By action of touching the oil-based cosmetic applied to the skin, a dynamic external force is applied to the cellulose powder, the viscosity is reduced, and good slippage is felt.

On the other hand, Patent Literatures 4 and 5 use minute cellulose, and particles used in these literatures are manufactured in a state of excessively containing water. In this case, a manufactured cosmetic may be excessively emulsified because the cosmetic inevitably contains water. Patent Literature 6 discloses that when a specific acylated cellulose derivative, a volatile hydrocarbon oil, and a nonvolatile linear silicone oil are used in combination at a specific ratio, an excellent smooth feeling is obtained, and stickiness does not occur immediately after application. The cellulose derivative is obtained by modifying hydroxyethyl cellulose or hydroxypropyl cellulose. This treatment is intended to improve solubility in an oil agent, and in this case, unlike the present invention, the cellulose derivative is not present in a particulate state in a composition.

In addition to the above aspect, the following aspect is also preferable.

### (Second Aspect)

The oil-based cosmetic according to the first aspect,
wherein the cellulose powder is formed of a composite containing the fine fibrous cellulose and a polyhydric alcohol.

### (Third Aspect)

The oil-based cosmetic according to the first aspect, further containing petrolatum.

### (Fourth Aspect)

The oil-based cosmetic according to the first aspect, having a B-type viscosity of 100 to 50,000 mPa·s under measurement conditions of 35°C and a rotation speed of 3 rpm.

### (Fifth Aspect)

The oil-based cosmetic according to the first aspect,
wherein the fluid oil has a melting point of -10°C to 25°C.

### (Sixth Aspect)

The oil-based cosmetic according to the first aspect, wherein the fluid oil is liquid paraffin.

### (Seventh Aspect)

The oil-based cosmetic according to the first aspect,
containing the cellulose powder in an amount of 1 to 20% by mass.

### (Eighth Aspect)

The oil-based cosmetic according to the first aspect,
wherein the cellulose powder has a moisture percentage of 1 to 20%.

### (Ninth Aspect)

The oil-based cosmetic according to the first aspect, wherein the cellulose powder has a loose bulk density of 100 to 1000 mg/cm³.

### (Tenth Aspect)

The oil-based cosmetic according to the first aspect, wherein the cellulose powder is white or light yellow.

### (Eleventh Aspect)

The oil-based cosmetic according to the first aspect, further including an additive.

### (Twelfth Aspect)

The oil-based cosmetic according to the second aspect, wherein the polyhydric alcohol is glycerin.

### Advantageous Effects of Invention

According to the present invention, there is provided an oil-based cosmetic that hardly imparts a foreign body feeling and suppresses stickiness and gloss.

### Brief Description of Drawings

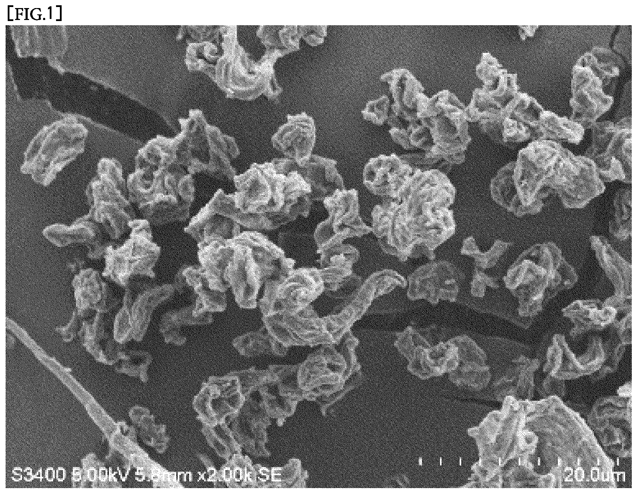

Fig. 1 is an SEM image of a spray dry powder.

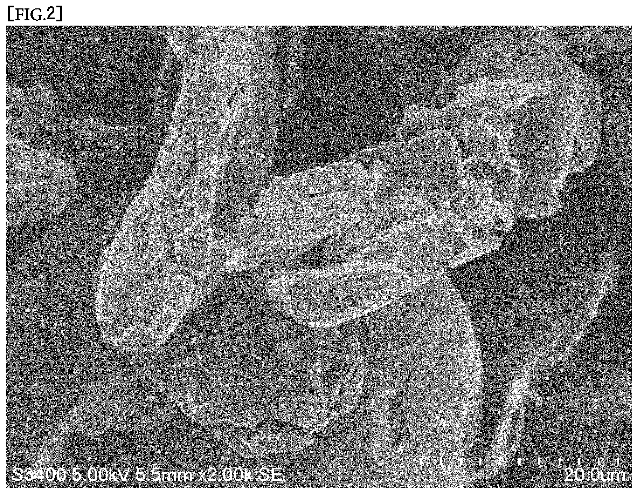

Fig. 2 is an SEM image of a drum dry powder.

### Description of Embodiments

An embodiment for carrying out the present invention will be described below. Note that the present embodiment is an example of the present invention. The scope of the present invention is not limited to the scope of the present embodiment.

An oil-based cosmetic according to the present embodiment contains a fluid oil and a cellulose powder, in which the cellulose powder contains dried fine fibrous cellulose having an average fiber diameter of 1 to 1000 nm, has an average particle size of 1 to 100 µm, and has a specific surface area of 0.5 to 10 m²/g. Before the oil-based cosmetic is described, the fine fibrous cellulose which is a raw material of the cellulose powder will be described.

### (Fine fibrous cellulose)

The fine fibrous cellulose can be obtained by defibrating a raw material pulp (making a raw material pulp finer), and can be manufactured by a known treatment method such as chemical treatment or mechanical treatment.

As the raw material pulp of the fine fibrous cellulose, one or more selected from the group consisting of, for example, a wood pulp made from hardwood, softwood, or the like, a non-wood pulp made from straw, bagasse, cotton, hemp, bast fiber, or the like, and a de-inked pulp (DIP) made from tea used paper, envelope used paper, magazine used paper, flyer used paper, corrugated used paper, high-quality white used paper, simili used paper, wood-free used paper, recovered used paper, waste paper, or the like can be used. Note that the above various raw materials may be, for example, in a state of a ground product. In recent years, a demand for organic component-containing products in consideration of environmental load reduction tends to increase, and thus a plant-derived wood pulp made from hardwood or softwood other than used paper is particularly suitable.

As the wood pulp, one or more selected from, for example, the group consisting of a chemical pulp such as a hardwood kraft pulp (LKP), a softwood kraft pulp (NKP), a sulfite pulp (SP), or a dissolving pulp (DP), and a mechanical pulp (TMP) can be used. In particular, a chemical pulp such as a hardwood kraft pulp (LKP) or a softwood kraft pulp (NKP), which is a wood pulp that enhances a cellulose component, is preferable, and a bleached pulp (BKP) is suitable.

As the mechanical pulp, for example, one or more selected from the group consisting of a stone ground pulp (SGP), a pressure stone ground pulp (PGW), a refiner ground pulp (RGP), a chemiground pulp (CGP), a thermoground pulp (TGP), a ground pulp (GP), a thermomechanical pulp (TMP), a chemithermomechanical pulp (CTMP), a refiner mechanical pulp (RMP), and a bleached thermomechanical pulp (BTMP) can be used.

It is preferable to use a kraft pulp which is easily defibrated and has high dispersibility from a viewpoint of manufacturing fine fibrous cellulose having a relatively small average fiber diameter. In particular, in a case of application to a white or light yellow product (for example, an oil-based cosmetics), it is convenient when the fine fibrous cellulose itself is white, and it is more preferable to use LBKP and NBKP from a viewpoint of improving the level of whiteness.

The fine fibrous cellulose may be subjected to a pretreatment prior to defibration. For example, as the pretreatment, the raw material pulp may be mechanically pre-beaten, or the raw material pulp may be chemically modified. A method for pre-beating the raw material pulp is not particularly limited, and a known method can be used.

Examples of the pretreatment of the raw material pulp by a chemical method include hydrolysis (acid treatment) of a polysaccharide with an acid (for example, sulfuric acid), hydrolysis (enzyme treatment) of a polysaccharide with an enzyme, swelling of a polysaccharide with an alkali (alkali treatment), oxidation (oxidation treatment) of a polysaccharide with an oxidizing agent (for example, ozone), reduction (reduction treatment) of a polysaccharide with a reducing agent, oxidation (oxidation treatment) with a TEMPO catalyst, anionization (anion treatment) by phosphoric acid esterification, carbamate formation, or the like, and cationization (cation treatment).

Examples of an alkali used for the alkali treatment include an organic alkali such as sodium hydroxide, lithium hydroxide, potassium hydroxide, an ammonia aqueous solution, tetramethylammonium hydroxide, tetraethylammonium hydroxide, tetrabutylammonium hydroxide, or benzyltrimethylammonium hydroxide. Sodium hydroxide is preferably used from a viewpoint of manufacturing cost.

When an enzyme treatment, an acid treatment, or an oxidation treatment is performed, the water retention degree of the fine fibrous cellulose can be lowered, the degree of crystallinity can be increased, and homogeneity can be increased. When the water retention degree of the fine fibrous cellulose is low, the fine fibrous cellulose is easily dehydrated and easily dried. Therefore, aggregation of the fine fibrous cellulose is promoted in manufacturing the cellulose powder by a method of freezing/drying under reduced pressure, which is preferable.

When an enzyme treatment, an acid treatment, or an oxidation treatment is performed on the raw material pulp, an amorphous region of hemicellulose or cellulose included in the pulp is decomposed. As a result, energy for making the raw material pulp finer can be reduced, and uniformity and dispersibility of cellulose fibers can be improved. The dispersibility of the cellulose fibers contributes to, for example, improvement of homogeneity of the average particle size of the cellulose powder. Note that it is preferable to avoid excessive pretreatment because the pretreatment reduces an axial ratio of the fine fibrous cellulose.

Examples of the fine fibrous cellulose modified by introduction of an anionic functional group by anionization include fine fibrous cellulose esterified with phosphorus-oxo acid, fine fibrous cellulose carbamated, and fine fibrous cellulose in which a hydroxy group of a pyranose ring is directly oxidized to a carboxyl group.

The fine fibrous cellulose modified by introduction of an anionic functional group has relatively high dispersibility. This is presumed to be because charge bias is locally generated by the anionic functional group, and this anionic functional group easily forms a hydrogen bond with water or an organic solvent in a dispersion.

When esterification with a phosphorus-oxo acid, which is an example of anionization, is performed on cellulose fibers, a fiber raw material can be made finer, and fine fibrous cellulose to be manufactured has a large axial ratio, an excellent strength, a high light transmittance, and a high viscosity. The esterification with phosphorus-oxo acid can be performed by a method described in JP 2019-199671 A. Modified fine fibrous cellulose into which a phosphorous acid ester group is introduced by modifying a hydroxy group of cellulose fibers can be exemplified.

Cellulose fibers can be defibrated by the following defibrating apparatus or method. The defibration can be performed, for example, by using one or more means selected from a homogenizer such as a high-pressure homogenizer or a high-pressure homogenizing apparatus, a stone mill type friction machine such as a grinder or a grinding machine, a refiner such as a conical refiner or a disc refiner, and various bacteria. Note that the cellulose fibers are preferably defibrated using an apparatus or method for making the cellulose fibers finer by a water flow, particularly by a high-pressure water flow. According to this apparatus or method, dimensional uniformity and dispersion uniformity of fine fibrous cellulose to be obtained are very high. In contrast, for example, when a grinder that performs grinding between rotating grindstones is used, it is difficult to make the cellulose fibers finer uniformly, and a fiber mass that cannot be defibrated may partly remain in some cases.

Examples of a grinder used for defibrating the cellulose fibers include Masscolloider manufactured by Masuko Sangyo Co., Ltd. Examples of an apparatus for making the cellulose fibers finer by a high-pressure water flow include Starburst (registered trademark) manufactured by Sugino Machine Co., Ltd. and Nanovater (registered trademark) manufactured by Yoshida Machine Industry Co., Ltd. Examples of a high-speed rotary homogenizer used for defibrating the cellulose fibers include CLEARMIX-11S manufactured by M Technique Co., Ltd.

The present inventors found that cellulose fibers defibrated by a method for making the cellulose fibers finer by a high-pressure water flow had a more uniform fiber width than cellulose fibers defibrated by a method for performing grinding between rotating grindstones when the present inventors observed the obtained fibers with a microscope.

Defibration by a high-pressure water flow is suitably performed by pressurizing a dispersion of the cellulose fibers with a pressure intensifier, for example, to 30 MPa or more, preferably 100 MPa or more, more preferably 150 MPa or more, particularly preferably 220 MPa or more (high pressure condition), ejecting the dispersion from a nozzle having a pore diameter of 50 µm or more, and reducing the pressure such that a pressure difference is, for example, 30 MPa or more, preferably 80 MPa or more, and more preferably 90 MPa or more (pressure reduction condition). Pulp fibers are defibrated by a cleavage phenomenon caused by this pressure difference. When a pressure under the high pressure condition is low, or when a pressure difference from the high pressure condition to the pressure reduction condition is small, defibration efficiency decreases, and it is necessary to repeatedly perform defibration (eject the dispersion from the nozzle) in order to obtain a desired fiber width.

As an apparatus for defibration by a high-pressure water flow, a high-pressure homogenizer is preferably used. The high-pressure homogenizer refers to a homogenizer capable of ejecting a slurry of cellulose fibers at a pressure of, for example, 10 MPa or more, preferably 100 MPa or more. When the cellulose fibers are treated with a high-pressure homogenizer, collisions between the cellulose fibers, a pressure difference, microcavitation, and the like occur, and the cellulose fibers are effectively defibrated. Therefore, the number of times of defibration treatment can be reduced, and manufacturing efficiency of the fine fibrous cellulose can be increased.

As the high-pressure homogenizer, a high-pressure homogenizer that causes counter collision of slurry flows of the cellulose fibers on a straight line is preferably used. Specific examples of the high-pressure homogenizer include a counter collision type high-pressure homogenizer (MICROFLUIDIZER (registered trademark), wet jet mill). In this apparatus, two upstream flow paths are formed such that the pressurized slurry flows of the cellulose fibers cause counter collision with each other at a junction. The slurry flows of the cellulose fibers cause collision at the junction, and the slurry of the cellulose fibers that has caused collision flows out of a downstream flow path. The downstream flow path is formed perpendicular to the upstream flow paths, and the upstream flow paths and the downstream flow path form a T-shaped flow path. By using such a counter collision type high-pressure homogenizer, energy imparted from the high-pressure homogenizer is converted into collision energy to the maximum. Therefore, the cellulose fibers can be more efficiently defibrated.

The fine fibrous cellulose obtained by fibrillation can be dispersed in a water-based medium and stored as a dispersion until used as a raw material of the cellulose powder. The entire water-based medium is particularly preferably water (aqueous dispersion). Note that a part of the water-based medium may be another liquid that is compatible with water. As the other liquid, for example, a lower alcohol having 3 or less carbon atoms can be used.

The fine fibrous cellulose forming the cellulose powder of the present embodiment may be made of only unmodified fine fibrous cellulose, may be made of only modified fine fibrous cellulose, or may contain unmodified fine fibrous cellulose and modified fine fibrous cellulose. Examples of the modified fine fibrous cellulose include fine fibrous cellulose obtained by modifying (replacing) a hydroxy group of cellulose with a functional group, for example, fine fibrous cellulose that has been subjected to TEMPO oxidation, fine fibrous cellulose that has been subjected to phosphorous acid esterification, or fine fibrous cellulose carbamated.

When the cellulose powder is made of modified fine fibrous cellulose, a dispersion obtained by dispersing the cellulose powder in a dispersion medium exhibits a transparent color. On the other hand, when the cellulose powder is made of unmodified fine fibrous cellulose, a dispersion obtained by dispersing the cellulose powder in a dispersion medium exhibits white or light yellow. By adjusting a ratio between modified fine fibrous cellulose and unmodified fine fibrous cellulose in the fine fibrous cellulose forming the cellulose powder, a dispersion having an intermediate color between a white color and a transparent color can be manufactured.

The cellulose powder is white or light yellow regardless of whether the raw material is modified or unmodified fine fibrous cellulose. Since modified fine fibrous cellulose has an average fiber diameter smaller than unmodified fine fibrous cellulose, when comparison is made between cellulose powders having the same mass, a cellulose powder made of fine fibrous cellulose containing the modified fine fibrous cellulose tends to have a larger specific surface area than a cellulose powder made of only the unmodified fine fibrous cellulose.

The raw material pulp is preferably defibrated such that physical properties and the like of fine fibrous cellulose to be obtained have desired values or evaluations as described below.

### <Average fiber diameter>

An upper limit of the average fiber diameter (average fiber width. average diameter of single fibers.) of the fine fibrous cellulose is 1000 nm, preferably 500 nm or less, more preferably 100 nm or less, and particularly preferably 50 nm or less. When the average fiber diameter of the fine fibrous cellulose exceeds 1000 nm, the average particle size of a formed cellulose powder is relatively large. When the average particle size is too large, a foreign body feeling is strongly felt when the oil-based cosmetic is applied to the skin. A lower limit of the average fiber diameter of the fine fibrous cellulose is 1 nm, preferably 2 nm or more, and more preferably 3 nm or more. When the average fiber diameter of the fine fibrous cellulose is less than 1 nm, handleability is poor in manufacturing a cellulose powder because a dispersion of the fine fibrous cellulose has a high viscosity.

The average fiber diameter of the fine fibrous cellulose can be adjusted by, for example, selection of a raw material pulp, a pretreatment, or defibration.

A method for measuring the average fiber diameter of the fine fibrous cellulose is as follows.

First, 100 ml of an aqueous dispersion of fine fibrous cellulose having a solid concentration of 0.01 to 0.1% by mass is filtered through a Teflon (registered trademark) membrane filter, and solvent substitution is performed once with 100 ml of ethanol and three times with 20 ml of t-butanol. Next, the resulting product is freeze-dried and coated with osmium to obtain a sample. This sample is observed with an electron microscope SEM image at any magnification of 3,000 to 30,000 depending on the width of a fiber constituting the sample. Specifically, two diagonals are drawn on the observation image, and three straight lines passing an intersection of the diagonals are arbitrarily drawn. Furthermore, the widths of 100 fibers in total intersecting with the three straight lines are visually measured. Then, a median diameter of the measured values is taken as an average fiber diameter.

### <Average fiber length>

The average fiber length (average length of single fibers) of the fine fibrous cellulose is, for example, preferably 0.01 to 1000 µm, and more preferably 0.03 to 500 µm. When the average fiber length exceeds 1000 µm, fibers are likely to be entangled with each other when the fine fibrous cellulose is dried, and are less likely to be loosened when dispersed in an oil-based dispersion medium. In addition, another substance is easily carried by the fine fibrous cellulose, and a cellulose powder having functionality of the other substance is obtained. When the average fiber length is less than 0.01 µm, a cellulose powder having poor entanglement is obtained.

The average fiber length can be arbitrarily adjusted by, for example, selection of a raw material pulp, a pretreatment, or defibration.

In a method for measuring the average fiber length of the fine fibrous cellulose, similarly to the case of the average fiber diameter, the length of each fiber is visually measured. A median diameter of the measured values is taken as an average fiber length.

### <Axial ratio>

The fine fibrous cellulose has an axial ratio of preferably 10 to 1,000,000, more preferably 30 to 500,000. When the axial ratio is less than 10, the cellulose powder contained in the fluid oil may be easily loosened into individual fine fibrous cellulose. On the other hand, when the axial ratio is more than 1,000,000, the cellulose powder has an extremely large average particle size, and when the oil-based cosmetic is applied to the skin, a gritty feeling (foreign body feeling) may be felt.

### <Degree of crystallinity>

A lower limit of the degree of crystallinity of the fine fibrous cellulose is preferably 50, more preferably 60, and particularly preferably 70, and an upper limit thereof is preferably 100, more preferably 95, and particularly preferably 90. When the degree of crystallinity is less than 50, entanglement of fibers is weak due to an influence of temperature change or the like during drying, power of holding another substance is weak, and it is difficult to form a cellulose powder having a desired particle size.

The degree of crystallinity is a value measured by an X-ray diffraction method conforming to JIS-K0131 (1996) "General rules for X-ray diffraction analysis". Note that the fine fibrous cellulose has an amorphous portion and a crystalline portion, and the degree of crystallinity means a ratio of the crystalline portion in the entire fine fibrous cellulose.

### <Pseudo particle size distribution>

A pseudo particle size distribution curve of the fine fibrous cellulose preferably has one peak. In the case of one peak, the fiber length and the fiber diameter of the fine fibrous cellulose have high uniformity, and entanglement between fibers of the fine fibrous cellulose easily occurs when the cellulose powder is manufactured. Therefore, the cellulose powder is less likely to be loosened in the fluid oil. In addition, the cellulose powder has a small statistical variation in particle size. When the cellulose powder carries inorganic fine particles, the cellulose powder is sufficiently dispersed in the fluid oil and diffuses and reflects incident light. Therefore, the skin to which the oil-based cosmetic is applied has less gloss.

A peak value in the pseudo particle size distribution curve of the fine fibrous cellulose is measured conforming to ISO-13320 (2009). More specifically, a volume-based particle size distribution of the fine fibrous cellulose in an aqueous dispersion is examined using a particle size distribution measuring apparatus (a laser diffraction/scattering type particle size distribution measuring instrument manufactured by Seishin Enterprise Co., Ltd.). Then, the most frequent diameter of the fine fibrous cellulose is measured from this distribution. This most frequent diameter is taken as the peak value. The fine fibrous cellulose preferably has a single peak in a pseudo particle size distribution curve measured by a laser diffraction method in a water-dispersed state. As described above, the fine fibrous cellulose having one peak is sufficiently made finer and can exhibit good physical properties as the fine fibrous cellulose, which is preferable. Note that the peak value of the particle size of the fine fibrous cellulose in the pseudo particle size distribution, which is the single peak, is, for example, preferably 300 µm or less, more preferably 200 µm or less, and particularly preferably 100 µm or less. When the peak value exceeds 300 µm, there are many relatively large fibers, a variation in the particle size of the cellulose powder is large, and the shape of the cellulose powder tends to be nonuniform.

The peak value and the median diameter in the pseudo particle size distribution curve of the fine fibrous cellulose can be adjusted by, for example, selection of a raw material pulp, a pretreatment, or defibration.

### <Water retention degree>

The water retention degree of the fine fibrous cellulose is not particularly limited, but for example, unmodified fine fibrous cellulose has a water retention degree of 500% or less, more preferably 100 to 500%. When the water retention degree is more than 500%, the fine fibrous cellulose itself has a high water retention capacity and a poor dehydrating property. Therefore, even when the fine fibrous cellulose is manufactured through a drying process, drying time is long and productivity is deteriorated. A lower limit of the water retention degree of the fine fibrous cellulose is not particularly limited, but when the water retention degree is 100% or more, a binding force between fibers of the fine fibrous cellulose acts, and the shape of the cellulose powder is easily maintained.

The water retention degree of the fine fibrous cellulose can be arbitrarily adjusted by, for example, selection of a raw material pulp, a pretreatment, or defibration.

The water retention degree of the fine fibrous cellulose is a value measured conforming to JAPAN TAPPI No. 26 (2000).

### <Pulp viscosity>

The fibrillated fine fibrous cellulose has a pulp viscosity of 1 to 10 mPa·s, more preferably 2 to 9 mPa·s, particularly preferably 3 to 8 mPa·s. The pulp viscosity is the viscosity of a solution obtained by dissolving cellulose in a copper ethylenediamine solution. The larger the pulp viscosity, the higher the degree of polymerization of cellulose, which also affects the strength of a fiber itself.

### <Polyhydric alcohol>

A polyhydric alcohol can be added for the purpose of improving dispersibility of the cellulose powder manufactured through the drying process in the fluid oil. This is because the bulk specific gravity of the cellulose powder is smaller than the specific gravity of the fluid oil. However, the powder itself may settle down in a lower part of the fluid oil depending on the specific gravity of the cellulose itself. Such sedimentation of the powder may form secondary particles or tertiary particles due to aggregation of particles of the powder. In order to prevent this, the cellulose powder is preferably formed of a composite containing the fine fibrous cellulose and a polyhydric alcohol. As a result, it is considered that a state in which the cellulose powder is dispersed in the fluid oil is maintained, and the cellulose powder is less likely to be unevenly distributed. The polyhydric alcohol itself does not deteriorate the degree of stickiness, and has no effect of promoting gloss. A blending ratio of the polyhydric alcohol to the fine fibrous cellulose (= polyhydric alcohol : fine fibrous cellulose (based on solid content)) is 50 : 50 to 10 : 90, and preferably 40 : 60 to 20 : 80. When a blending ratio of the polyhydric alcohol to the fine fibrous cellulose is excessively large, a sticky dry product (cellulose powder) is obtained, a lightweight feeling of the cellulose powder of the present invention is lost, and handleability is deteriorated. On the other hand, when the blending ratio is excessively small, the dispersion effect may be deteriorated.

Examples of the polyhydric alcohol include a polyhydric alcohol having 2 to 6 carbon atoms and 2 or 3 oxygen atoms. Specifically, glycerin, propylene glycol, butylene glycol, pentanediol, dipropylene glycol, hexanediol, heptanediol, ethylene glycol, diethylene glycol, 1,3-propanediol, 3-methyl -1, 3-butanediol, and the like can be used, but are not limited thereto. In particular, glycerin is preferable from a viewpoint of a thickening property and dispersibility of composite particles.

### <Inorganic fine particles>

The cellulose powder composite may contain inorganic fine particles in addition to the fine fibrous cellulose and the polyhydric alcohol. The inorganic fine particles can impart various functions to the cellulose powder, and for example, by imparting metallic inorganic fine particles, an effect of diffusing and reflecting incident light can be expected. An oil-based cosmetic containing a cellulose powder containing inorganic fine particles diffuses and reflects incident light, and thus suppresses gloss of an application portion.

An upper limit of the content percentage of the inorganic fine particles in the cellulose powder is preferably 40% by mass, and the content percentage is preferably 30% by mass or less. A lower limit of the content percentage of the inorganic fine particles is preferably 0% by mass, and the content percentage is preferably 5% by mass or more. When the content percentage exceeds 50% by mass, the specific gravity of the cellulose powder is large due to inclusion of the inorganic fine particles. Therefore, sedimentation in the oil-based cosmetic may be promoted to impair dispersibility. On the other hand, when the content percentage is 5% by mass or more, the effect of diffusing and reflecting incident light is sufficiently exhibited.

An upper limit of the primary particle size of the inorganic fine particles is preferably 10 µm, and the primary particle size is preferably 5 µm or less, and more preferably 1 µm or less. When the primary particle size of the inorganic fine particles exceeds 10 µm, the inorganic fine particles are less likely to be carried by the fine fibrous cellulose. In addition, the surface area of the cellulose powder is not sufficiently large. A lower limit of the primary particle size of the inorganic fine particles is not particularly limited, but is preferably 1 nm, and the primary particle size is preferably 2 nm or more, and more preferably 3 nm or more. In a case where the primary particle size of the inorganic fine particles is 1 nm or more, when the inorganic fine particles are mixed with a raw material slurry for manufacturing the cellulose powder, the inorganic fine particles are likely to be dispersed in and cling to the fine fibrous cellulose.

A method for measuring the primary particle size of the inorganic fine particles can be performed by electron microscope observation, and an average value of the obtained particle sizes is taken as a measured value.

The inorganic fine particles can of course be used as they are, but when the inorganic fine particles are subjected to a hydrophilic treatment, the inorganic fine particles are easily acclimated to a raw material slurry for manufacturing the cellulose powder, which is preferable. A surface treatment agent used for the hydrophilic treatment has an effect of suppressing surface activity of the inorganic fine particles, improving dispersibility of the inorganic fine particles, and improving transparency and creakiness. The surface treatment agent for the inorganic fine particles is not particularly limited as long as it is a treatment agent capable of being dispersed in the raw material slurry, but a surface treatment agent containing anhydrous silicic acid or hydrous silicic acid is preferable.

The inorganic fine particles are not particularly limited, and known inorganic fine particles can be used. Examples thereof include barium titanate, lead zirconate titanate, silicon carbide, silicon nitride, aluminum nitride, alumina, zirconia, zircon, titanium oxide, zinc oxide, iron oxide, and cerium oxide. The cellulose powder containing these powders and the fine fibrous cellulose is preferable because of having excellent redispersibility in a liquid. For example, one or a combination of two or more selected from the group consisting of titanium oxide, zinc oxide, iron oxide, and cerium oxide can be used from a viewpoint of suppressing transmission of sunlight. In particular, when the inorganic fine particles are made of titanium oxide, a rutile type is preferable because transmission suppression of incident light in the oil-based cosmetic is improved.

The shapes of the inorganic fine particles that can be contained in the cellulose powder are not particularly limited, but examples thereof include a spherical shape, a rod shape, a needle shape, a spindle shape, a plate shape, and a polygonal shape.

The inorganic fine particles may be attached to a surface of the fine fibrous cellulose in the cellulose powder, or may be encapsulated in the fine fibrous cellulose. When the inorganic fine particles are encapsulated in the fine fibrous cellulose, the inorganic fine particles can be carried not only on a surface of the cellulose powder but also inside the cellulose powder, and even when the cellulose powder is irradiated with incident light from various angles, the cellulose powder diffuses and reflects the incident light, which is preferable. Here, the encapsulation can be said to be a state in which a part of a surface of the inorganic fine particles is covered with the fine fibrous cellulose, or a state in which the inorganic fine particles are covered with the fine fibrous cellulose and cannot be observed when the cellulose powder is observed from the outside.

The inorganic fine particles can be added to the fine fibrous cellulose before being dried, and it is preferable to mix the inorganic fine particles and the fine fibrous cellulose so as to be uniform.

### (Cellulose powder)

The cellulose powder of the present embodiment is formed by drying the fine fibrous cellulose. However, when viewed microscopically, the cellulose powder of the present embodiment includes a cellulose powder formed by drying a single fiber of the fine fibrous cellulose as it is (for example, a cellulose powder formed by entanglement of one yarn in the yarn or a cellulose powder formed by drying up a single fiber of the fine fibrous cellulose), and a cellulose powder formed by aggregation of a plurality of fibers of the fine fibrous cellulose during drying to form an aggregated mass. The fine fibrous cellulose is manufactured from a raw material pulp, and when dried, the fibers are wrinkled and shrunk. Therefore, a cellulose powder to be formed has a shape that is difficult to express but has irregularities. For example, the cellulose powder has a shape in which dried up fibers of fine fibrous cellulose are aggregated, the shape of Kompeito, or a shape formed by crumpling and rolling one or more sheets of Japanese writing paper or the like. The cellulose powder has a white color, a light yellow color, a cream color, a light orange color, or a mixed color of these colors. In particular, a white or light yellow cellulose powder is not conspicuous even when mixed with the fluid oil, which is preferable.

Since the cellulose, which is a constituent unit of the fine fibrous cellulose, has a hydroxy group (OH group) and a hydrogen group (H group), the cellulose powder containing the fine fibrous cellulose also has a hydroxy group and a hydrogen group. A hydroxy group or a hydrogen group is hydrogen-bonded to another hydroxy group or hydrogen group, whereby a molecule of the fine fibrous cellulose causes hydrogen bonding in the molecule of the cellulose or molecules of the fine fibrous cellulose are hydrogen-bonded to each other to form a three-dimensional network structure of the cellulose powder. When the cellulose powder is mixed with a water-based medium, the hydrogen bond is loosened due to hydrolysis or the like, aggregation of the cellulose is weakened, and the cellulose powder and the loosened fine fibrous cellulose are dispersed in the water-based medium. On the other hand, when the cellulose powder is mixed not with a water-based medium but with an oil-based medium, the following is presumed. Since the oil-based medium is hydrophobic, it is more difficult for the cellulose powder to be loosened in the oil-based medium than in the water-based medium. The cellulose powder is dispersed in the oil-based medium having a certain viscosity while maintaining the shape of the cellulose powder. In this case, the cellulose powder hardly settles down and maintains a dispersed state.

The cellulose powder according to the present embodiment contains the fine fibrous cellulose in an amount of preferably 50% by mass or more, more preferably 60% by mass or more, still more preferably 70% by mass or more, and an upper limit thereof may be 100% by mass. When the mass percentage of the fine fibrous cellulose in the cellulose powder is less than 50% by mass, there is a possibility that the desired bulk density and specific surface area of the cellulose powder of the present invention cannot be obtained.

### <Average particle size>

The cellulose powder according to the present embodiment has an average particle size in a range of preferably 1 to 100 µm, more preferably 1 to 70 µm, still more preferably 1 to 30 µm. The effect of the present invention is exhibited even when the average particle size is less than the above range, but it is desirable that the average particle size is equal to or more than the lower limit of the above range from a viewpoint of ease of handling. On the other hand, when the average particle size exceeds the above range, a void formed between particles is large when the cellulose powder is filled or dispersed in a dispersion medium, and it is difficult to adjust a concentration to a desired concentration.

The cellulose powder has a standard deviation of particle sizes of preferably 1 to 90 µm, more preferably 1 to 60 µm, particularly preferably 1 to 30 µm.

The cellulose powder of the present embodiment is characterized by having various particle sizes. Specifically, a dispersion coefficient of the particle sizes of the cellulose powder is statistically larger than a dispersion coefficient of particle sizes of inorganic fine particles. Note that the cellulose powder is not excellent in sphericity, and particles thereof have irregularities, porous shapes, and different shapes from each other.

The cellulose powder of the present embodiment has a porous shape. Each pore constituting the porous shape has, for example, a pore size of 0.1 nm to 2000 nm. When the cellulose powder carries inorganic fine particles, the inorganic fine particles may be stuck in the pores or may be attached to a surface of the cellulose powder. Therefore, the inorganic fine particles can be carried by the cellulose powder regardless of the sizes of the pores. Note that not only the inorganic fine particles but also a fluid oil, a surfactant, and an additive can be carried by the cellulose powder.

In addition, since the cellulose powder has a porous shape, the pores reflect incident light from the outside at various angles. Therefore, the oil-based cosmetic containing the cellulose powder has an effect of suppressing gloss.

Furthermore, since the cellulose powder of the present embodiment is manufactured by, for example, drying, a plurality of dried up and wrinkled fibers of the fine fibrous cellulose may be entangled to form porous pores. There are various ways of drying up the fine fibrous cellulose, and a cellulose powder to be formed contains not particles having a single shape but particles having various shapes.

An average particle size, a median diameter, a cumulative 10% diameter, and a cumulative 90% diameter of the cellulose powder are numerical values measured by a dry method using a measuring apparatus conforming to ISO-13320 (2009), specifically, a laser diffraction/scattering type particle size distribution measuring apparatus (particle size distribution) "LA-960V2" without splashing off moisture attached to the cellulose powder.

### <Specific surface area>

The cellulose powder has a specific surface area of preferably 0.1 to 10 m²/g, more preferably 0.2 to 8 m²/g, still more preferably 0.5 to 5 m²/g. When the specific surface area is less than 0.1 m²/g, the amount of inorganic fine particles that can be carried is small when the inorganic fine particles are to be carried by the cellulose powder, and there is a possibility that performance of the inorganic fine particles cannot be exhibited. On the other hand, a cellulose powder having a specific surface area of more than 10 m²/g is preferable from a viewpoint of weight reduction of particles and redispersibility, but is very difficult to manufacture.

The specific surface area of the cellulose powder was measured by a BET method. Specifically, measurement was performed by an adsorption method with nitrogen gas using NOVA4200e manufactured by Quantachrome Instruments as a measuring instrument. The measurement was performed conforming to a test method of JISZ8830:2013.

### <Moisture percentage>

The cellulose powder has a moisture percentage of preferably 30% or less, more preferably 20% or less, still more preferably 15% or less. A cellulose powder having a moisture percentage exceeding 30% contains a large amount of moisture, and may be divided into a cellulose powder phase and a fluid oil phase when the oil-based cosmetic is left for a long time.

The cellulose powder according to the present embodiment has a loose bulk density of preferably 100 to 1000 mg/cm³, more preferably 100 to 900 mg/cm³, still more preferably 150 to 800 mg/cm³. A cellulose powder having a loose bulk density exceeding 1000 mg/cm³ forms an aggregate in which fibers are firmly entangled with each other in a similar manner to the above-described tight bulk density, has no elasticity, and imparts a hard use feeling. In addition, even when the cellulose powder is dispersed in an oil-based dispersion medium, the cellulose powder easily settles down, and it cannot be said that the cellulose powder has excellent dispersibility. A cellulose powder having a tight bulk density of less than 100 mg/cm³ can solve the above-described problem, but particles thereof are easily disintegrated, and therefore the cellulose powder is easily disintegrated when dispersed in a solvent.

The loose bulk density is one of items used for calculating a flowability index of Carr, and was measured conforming to an ASTM D6393-99 compressibility measurement method. The measuring instrument is a "multifunctional powder physical property measuring instrument Multitester MT-02" (manufactured by Seishin Enterprise Co., Ltd.).

### <Additive>

An additive can be added to the oil-based cosmetic. The additive is added for the purpose of preventing the skin from drying, imparting moisture to the skin, and the like. As the additive, either one or both of a polysaccharide and a water-retaining polymer can be exemplified, but the additive is not limited thereto. Examples of the polysaccharide include quince seed, veegum, xanthan gum, and a hyaluronic acid salt, but are not limited thereto. In particular, a hyaluronic acid salt or the like is preferable from a viewpoint of a thickening property and dispersibility of the cellulose powder. Examples of the water-retaining polymer include polyvinyl alcohol, polyvinyl pyrrolidone, a carboxyvinyl polymer, polyethylene glycol, a homopolymer or copolymer containing a monomer having a phosphorylcholine group as a constituent monomer, a homopolymer or copolymer containing a monomer having a sugar residue as a constituent monomer, and a homopolymer or copolymer containing a monomer having an amino acid residue as a constituent monomer. Specific examples thereof include a copolymer of an alkyl (meth)acrylate and polymethacryloyloxyethyl phosphorylcholine, a copolymer of an alkyl (meth)acrylate and methacryloyloxyethyl glucoside, and a copolymer of an alkyl (meth)acrylate and methacryloyl-L-lysine, but are not limited thereto. In particular, polyvinylpyrrolidone is preferable from a viewpoint of a thickening property and dispersibility of the cellulose powder.

### (Manufacture of cellulose powder: drum-drying method)

The cellulose powder is obtained by drying fine fibrous cellulose. Particles of the dried cellulose powder (dry body) are less likely to aggregate with each other, and are easily dispersed when put into a water-based or oil-based medium. Examples of the cellulose powder used in the present embodiment include a cellulose powder obtained by a drum-drying method and a cellulose powder obtained by a spray-drying method. According to a method for manufacturing the cellulose powder by the drum-drying method, even from fine fibrous cellulose having a relatively high concentration or poor fluidity, a dry body in which particles are hardly aggregated and easily dispersed can be obtained. The manufacture of the cellulose powder by the drum-drying method is performed as follows as an example.

The fine fibrous cellulose can be supplied to, for example, a drum dryer that performs a drying treatment in a slurry (aqueous dispersion) state, and the content (% by absolute dry mass) of the fine fibrous cellulose in this case is 1% by mass or more, preferably 1.5% by mass, and more preferably 2.0% by mass. The content is 10% by mass or less, preferably 7% by mass, and more preferably 5% by mass. When the content exceeds 10% by mass, a viscosity of the slurry is too high and a handling property is poor. On the other hand, when the content is less than 1% by mass, much energy and time are consumed to remove moisture, which is not economical.

The drum dryer used in the drying treatment by the drum-drying method may be a known drum dryer. For example, "John Millder JM-T type" manufactured by Johnson Boiler Co., Ltd. can be used. As the drum dryer, an inner-roll-type drum dryer can be suitably used. The inner-roll-type drum dryer performs a gentle drying treatment to provide a dry body having a relatively small specific surface area. The drying treatment can be performed under normal pressure.

Regarding operating conditions of the drum dryer, a surface temperature of an inner surface of a drum is 80 to 200°C, and preferably 90 to 190°C. At this surface temperature, a dry body (powder) suitable for the oil-based cosmetic can be obtained. When the surface temperature exceeds 200°C, some of fibers of the fine fibrous cellulose may be thermally modified. On the other hand, when the surface temperature is lower than 80°C, not only it takes a lot of time to remove moisture but also a powder having a very large amount of moisture is obtained. In addition, a rotation speed of the drum dryer can be, for example, 1 rpm or more and 2 rpm or less although it depends on the inner diameter of the drum and the amount of the slurry to be put. Time for drying with the drum dryer depends on the amount of the slurry to be put, but if the time is 1 second to 60 seconds, drying is sufficiently performed, and even if drying is performed for a longer time, the water content of a dry body does not decrease any more.

### (Manufacture of cellulose powder: spray-drying method)

The cellulose powder is manufactured by a spray-drying method as follows, for example. A spray dryer sprays a slurry from a spray nozzle included in the spray dryer into a drier can body to form droplets. The sprayed droplets of the slurry are exposed to hot air flowing in the drier can body and dried to form a dry body. In the present embodiment, the type of the spray dryer is not particularly limited, but for example, a product "TR-160" manufactured by PRECI, CO. LTD. can be used.

A spray pressure of the slurry is preferably 0.3 MPa or more, and more preferably 0.3 to 2.0 MPa. When the spray pressure is less than 0.3 MPa, a variation in the particle size of the obtained dry body is likely to occur.

A temperature of the hot air to be blown into the spray dryer is preferably adjusted in consideration of a discharged air temperature, and is preferably 180 to 220°C, and more preferably 190 to 220°C. When the temperature of air discharged from the spray dryer is 90 to 120°C, a sufficiently dried dry body is obtained, which is suitable.

The slurry to be dried may be a mixed slurry in which a polyhydric alcohol is mixed with water in addition to the fine fibrous cellulose. A dry body (cellulose powder) obtained by drying the mixed slurry is a composite containing the fine fibrous cellulose and the polyhydric alcohol, has a hydroxy group of the polyhydric alcohol in addition to a hydroxy group of the fine fibrous cellulose, and therefore exhibits better dispersibility in a medium.

Examples of the polyhydric alcohol include a dihydric alcohol, a trihydric alcohol, and another polyhydric alcohol. Examples of the dihydric alcohol include ethylene glycol, diethylene glycol, triethylene glycol, propylene glycol, and neopentyl glycol. Examples of the trihydric alcohol include glycerin, trimethylolethane, trimethylolpropane, and cyclohexanedimethanol. Examples of the polyhydric alcohol other than the dihydric and trihydric alcohols include pentaerythritol, diglycerin, and polyglycerin. These may be used singly or in combination of two or more kinds thereof. In particular, the trihydric alcohol has a high ratio of hydroxy groups in one molecule, and improves dispersibility of the dry body with a small amount thereof, which is preferable. Among the trihydric alcohols, glycerin is suitable.

The amount of the polyhydric alcohol is 5 parts by mass or more, preferably 10 parts by mass or more, and more preferably 15 parts by mass or more with respect to 100 parts by mass of the fine fibrous cellulose, and is 100 parts by mass or less, preferably 90 parts by mass or less, and more preferably 80 parts by mass or less with respect to 100 parts by mass of the fine fibrous cellulose. When the amount of the polyhydric alcohol is insufficient, there is a possibility that dispersibility is not improved. On the other hand, when the amount of the polyhydric alcohol is too large, a sticky feeling of the oil-based cosmetic is increased, which may cause discomfort. Note that the polyhydric alcohol is hardly vaporized even when the drying treatment is performed, and a ratio between the mass of the fine fibrous cellulose and the mass of the polyhydric alcohol in the mixed slurry can be a ratio between the mass of the fine fibrous cellulose and the mass of the polyhydric alcohol in the dry body as it is.

In addition to the polyhydric alcohol, a reagent having a hydroxy group, for example, a hydroxy acid, a hydroxy acid salt, a glycerin derivative, or a combination thereof may be added to the slurry.

The cellulose powder can be manufactured by a method for freeze-drying cellulose nanofibers as a raw material, a method for drying cellulose nanofibers under reduced pressure, a method for heat-drying cellulose nanofibers (for example, drying cellulose nanofibers with a hot dryer or a drum dryer), or a method for spray-drying cellulose nanofibers. In particular, when the method for heat-drying cellulose nanofibers is used, a cellulose powder having a relatively small specific surface area or average particle size is obtained, and can be suitably used for a cosmetic containing a fluid oil as a component. When the cosmetic containing a fluid oil as a component contains particles having a relatively large specific surface area, the cosmetic easily imparts a foreign body feeling. On the other hand, when particles contained in the cosmetic containing a fluid oil as a component have a relatively small specific surface area or average particle size, the foreign body feeling is reduced.

In addition, the cellulose powder of the present invention can be manufactured by, for example, a method for performing heating and drying. The fine fibrous cellulose constituting the cellulose powder is dried, and therefore wrinkled or dried up to form pores. The cellulose powder contains the fine fibrous cellulose as a main component, and is therefore a porous body. When the cellulose powder is porous, another substance can be carried by a large number of pores formed in the cellulose powder. By making the cellulose powder carry another substance, properties of the other substance can be imparted to the cellulose powder.

### (Fluid oil)

The fluid oil is one of main components of the oil-based cosmetic of the present invention, and has effects of facilitating application of the oil-based cosmetic to the skin, improving spreading performance, moisturizing the skin, imparting moisture to the skin, and the like. The fluid oil refers to an oil having fluidity at normal temperature (for example, 15 to 30°C), and has a melting point of, for example, 25°C or lower, preferably -10 to 25°C. An oil having a high viscosity at normal temperature is difficult to apply to the skin. As the fluid oil, those used in the field of cosmetics can be selected and used, and examples thereof include a liquid ester oil (isopropyl myristate, 2-octyldodecyl myristate, cetyl 2-ethylhexanoate, diisostearyl malate, or pentaerythritol tetraethylhexanoate), a liquid wax (jojoba oil), a liquid hydrocarbon oil (liquid paraffin or squalane), a higher fatty acid (lauric acid or isostearic acid), a liquid fat and oil (olive oil, camellia oil, macadamia nut oil, or castor oil), a liquid higher alcohol (isostearyl alcohol or 2-octyldodecanol), methylpolysiloxane, methylphenylpolysiloxane, and methylhydrogenpolysiloxane. Among these oils, a liquid hydrocarbon oil (particularly liquid paraffin) is almost odorless and preferable.

The content percentage of the fluid oil in the oil-based cosmetic is, for example, 20% by mass or more, preferably 20 to 80% by mass, and more preferably 30 to 60% by mass. When the content percentage of the fluid oil in the oil-based cosmetic is less than 20% by mass, the oil-based cosmetic is poor in moisturizing and moisture-imparting effects.

### (Normal temperature solid oil)

Due to a change in a surrounding environment, for example, a change in room temperature or humidity, dispersibility of the cellulose powder in the fluid oil may be deteriorated. For example, the fluidity or viscosity of the oil-based cosmetic may change due to a change in room temperature, and as a result, the cellulose powder dispersed in the fluid oil may be unevenly distributed gradually. In order to suppress occurrence of this uneven distribution and to maintain the dispersion, it is preferable to add a material having a higher viscosity to the oil-based cosmetic. The material is preferably petrolatum (yellow petrolatum or white petrolatum), shea butter, carnauba wax, candelilla wax, beeswax, or Japan wax, and more preferably petrolatum from a viewpoint of skin safety and hygiene, but is not limited thereto.

Petrolatum is preferable also from a viewpoint of having compatibility with the fluid oil. The content percentage of petrolatum in the oil-based cosmetic is preferably 10 to 70% by mass, preferably 20 to 60% by mass, and more preferably 30 to 50% by mass. When the content percentage exceeds 70% by mass, the viscosity of the oil-based cosmetic is too high, it is difficult to apply the oil-based cosmetic, and a use feeling is poor. On the other hand, when the content percentage is less than 10% by mass, the effect of adding petrolatum is poor.

### (Oil-based cosmetic)

In the oil-based cosmetic of the present embodiment, the content of the cellulose powder is not particularly limited as long as a B-type viscosity satisfies 10 to 50,000 mPa·s under a measurement condition of a rotation speed of 3 rpm. However, when the cellulose powder is excessive or insufficient, there is a possibility that a sense of discomfort or strong stickiness may be felt when the oil-based cosmetic is applied to the skin. Therefore, the oil-based cosmetic contains the cellulose powder in an amount of preferably 0.1 to 10% by mass, more preferably 0.2 to 8% by mass, still more preferably 0.3 to 5% by mass.

The B-type viscosity (measurement conditions: rotation speed of 3 rpm, 35°C) of the oil-based cosmetic is preferably 100 to 50,000 mPa·s, more preferably 500 to 50,000 mPa·s, and still more preferably 1000 to 50,000 mPa·s. When the B-type viscosity of the oil-based cosmetic exceeds 50,000 mPa·s, it is difficult to apply and spread the oil-based cosmetic on the skin, and it is difficult to obtain the effect of the cellulose powder. On the other hand, when the B-type viscosity of the oil-based cosmetic is less than 100 mPa·s, the oil-based cosmetic applied to the skin easily drips, and it is difficult to apply the oil-based cosmetic only to a desired site.

The oil-based cosmetic of the present invention can maintain a state in which the hydrophilic cellulose powder is dispersed in the hydrophobic fluid oil in principle without adding a surfactant. This is presumed to be because the cellulose powder of the present embodiment has a relatively low bulk density, and the cellulose powder is bulky in the fluid oil.

The oil-based cosmetic of the present embodiment imparts an excellent use feeling, and one of factors that affect this use feeling is fluidity of the oil-based cosmetic. The fluidity is affected by the melting point of the oil-based cosmetic, and the melting point is 40°C or lower, preferably 10 to 40°C, and more preferably 10 to 36°C. When the melting point of the oil-based cosmetic exceeds 40°C, the oil-based cosmetic may be solidified and difficult to use depending on an outside air temperature (or an indoor temperature). In addition, when the oil-based cosmetic is easily solidified or liquefied depending on an outside air temperature (or an indoor temperature), the cellulose powder in the oil-based cosmetic is unevenly distributed depending on a storage state.

The oil-based cosmetic contains the fluid oil and the cellulose powder, and a percentage of the fluid oil contained in the oil-based cosmetic is preferably 0.1 to 20% by mass, and more preferably 0.2 to 15% by mass. When the percentage is less than 0.1% by mass, the content percentage of the cellulose powder is relatively low, and therefore the effect of suppressing gloss of the oil-based cosmetic is poor. On the other hand, when the percentage exceeds 20% by mass, the viscosity is too high, whereby spreading performance on the skin is impaired.

### (Surfactant)

In the oil-based cosmetic of the present embodiment, since the cellulose powder is well dispersed in the fluid oil, in principle, no surfactant may be contained. However, when the oil-based cosmetic is left for a long period of time, the cellulose powder may be unevenly distributed, and therefore a surfactant may be added within a range not impairing the viscosity of the oil-based cosmetic to emulsify the oil-based cosmetic. When the oil-based cosmetic is emulsified, the oil-based cosmetic can be dispersed with a known disperser (homogenizer or the like) to be emulsified. It is considered that the oil-based cosmetic containing a surfactant reduces electrostatic repulsion between the cellulose powder and the fluid oil, and as a result, a state in which the cellulose powder is dispersed in the fluid oil is maintained for a long period of time. A percentage of the surfactant in the oil-based cosmetic is 0.1 to 10% by mass, and preferably 0.2 to 5% by mass.

As the surfactant, for example, a nonionic surfactant, an anionic surfactant, a cationic surfactant, an amphoteric surfactant, a phospholipid, and the like can be used. It is particularly preferable to use an ester type or ester-ether type nonionic surfactant, and examples thereof include a glycerin fatty acid ester, polyglycerin, a fatty acid ester, a propylene glycol fatty acid ester, a sorbitan fatty acid ester, a fatty acid ester of sorbitol, alkylene glycol adducts thereof, a polyalkylene glycol fatty acid ester, a sucrose fatty acid ester, polysorbate 20, polysorbate 60, polysorbate 80, a polyoxyalkylene alkyl ether, and a polyoxyethylene alkyl phenyl ether.

A material usually used in cosmetics, other than a surfactant and an additive, can also be added to the oil-based cosmetic as long as the material does not impair the viscosity.

### Examples

### <Test Example 1>

### 1. Manufacture of powder

Next, Examples will be described. Powder of Test Example 1 was manufactured as follows. A dispersion obtained by dispersing fine fibrous cellulose ("ELLEX (registered trademark) -S" manufactured by Daio Paper Corporation) in water so as to have a concentration of 2% by mass was supplied to a spray dryer as a raw material slurry to obtain cellulose powder A. The fine fibrous cellulose has an average fiber diameter of 50 nm.

### 2. Preparation of Test Example

White petrolatum (Kenei Pharmaceutical Co., Ltd., Japanese Pharmacopoeia) and liquid paraffin (FUJIFILM Wako Pure Chemical Corporation, Wako first class) were each weighed and taken, placed in a 100 ml container, mixed while being warmed with hot water at 90°C. An essential oil (orange oil, FUJIFILM Wako Pure Chemical Corporation, Wako first class) and the cellulose powder A were added thereto to adjust a blending ratio of the powder to 5%. Thereafter, the mixture was mixed and stirred (8000 rpm, 3 minutes) with a stirrer IKA-T25, and cooled to room temperature to obtain Test Example 1. Physical properties of the cellulose powder A, cellulose powder B described later, and inorganic fine particles are presented in Table 1.

**[Table 1]**

| | Unit | Cellulose powder A | Cellulose powder B | Titanium oxide |
|---|---|---|---|---|
| Loose bulk density | mg/cm³ | 369.0 | 226.0 | 735.0 |
| Specific surface area | m²/g | 1.9 | 1.0 | 96.0 |
| Average particle size | µm | 10.0 | 53.1 | 6.4 |
| Median diameter | µm | 9.4 | 47.6 | 8.1 |
| Cumulative 10% diameter | µm | 6.1 | 22.3 | 2.8 |
| Cumulative 90% diameter | µm | 14.8 | 88.3 | 13.7 |
| Moisture percentage | % | 4.7 | 9.5 | 1.2 |

### <Test Example 2>

### 1. Manufacture of powder

Test Example 2 was manufactured as follows. A dispersion obtained by dispersing fine fibrous cellulose in water so as to have a concentration of 2% by mass was supplied to a drum dryer as a raw material slurry to obtain cellulose powder B. The fine fibrous cellulose has an average fiber diameter of 50 nm.

### 2. Preparation of Test Example

White petrolatum and liquid paraffin were each weighed and taken, placed in a 100 ml container, and mixed while being warmed with hot water at 90°C. An essential oil and the cellulose powder B were added thereto to adjust a blending ratio of the powder to 5%. Thereafter, the mixture was mixed and stirred (8000 rpm, 3 minutes) with a stirrer IKA-T25, and cooled to room temperature to obtain Test Example 2.

### <Comparative Example 1>

### 1. Inorganic fine particles

In Comparative Example 1, titanium oxide (STR-100N, SAKAI CHEMICAL INDUSTRY CO., LTD.) as inorganic fine particles was prepared instead of the cellulose powder.

### 2. Preparation of Comparative Example

White petrolatum and liquid paraffin were each weighed and taken, placed in a 100 ml container, and mixed while being warmed with hot water at 90°C. An essential oil and the titanium oxide were added thereto to adjust a blending ratio of the titanium oxide to 5%. Thereafter, the mixture was mixed and stirred (8000 rpm, 3 minutes) with a stirrer IKA-T25, and cooled to room temperature to obtain Comparative Example 1.

### <Comparative Example 2>

### 1. Preparation of Comparative Example

In Comparative Example 2, no powder was added for comparison with Test Examples. Specifically, white petrolatum and liquid paraffin were each weighed and taken, placed in a 100 ml container, and mixed while being warmed with hot water at 90°C. An essential oil was added thereto to perform adjustment. Thereafter, the mixture was mixed and stirred (8000 rpm, 3 minutes) with a stirrer IKA-T25, and cooled to room temperature to obtain Comparative Example 2.

The moisture percentage and the B-type viscosity of each of the above-described Test Examples and Comparative Examples were measured. Measurement conditions of the B-type viscosity were a rotation speed of 3 rpm or 30 rpm and 35°C. Mixing ratios of the cellulose powder (or inorganic fine particles) and various reagents and measurement results of the B-type viscosity are presented in Table 2.

**[Table 2]**

| | Unit | Test Example 1 | Test Example 2 | Comparative Example 1 | Comparative Example 2 |
|---|---|---|---|---|---|
| White petrolatum | g | 25 | 25 | 25 | 25 |
| Liquid paraffin | g | 25 | 25 | 25 | 25 |
| Essential oil | g | 0.1 | 0.1 | 0.1 | 0.1 |
| Cellulose powder A | g | 2.6 | 0 | 0 | 0 |
| Cellulose powder B | g | 0 | 2.6 | 0 | 0 |
| Titanium oxide | g | 0 | 0 | 2.6 | 0 |
| Powder blending ratio | % | 5 | 5 | 5 | 0 |
| B-type viscosity (3 rpm, 35°C) | mPa·s | 7120 | 7020 | 7320 | 6390 |
| B-type viscosity (30 rpm, 35°C) | mPa·s | 1370 | 1439 | 1240 | 929 |

Here, the B-type viscosity was measured conforming to JIS-Z8803 (2011) "Method for measuring viscosity of liquid". The B-type viscosity is a resistance torque when a liquid is stirred, and a higher B-type viscosity means that more energy is required for stirring.

### (Sensory test)

A sensory test was performed using the prepared Test Examples and Comparative Examples. The contents of the sensory test are as follows. Test Examples and Comparative Examples were applied to the skins of subjects, and the subjects evaluated Test Examples and Comparative Examples for items at that time, specifically, for a foreign body feeling, stickiness, and gloss with any one of 1, 2, and 3 points, and for a use feeling with any one of first, second, and third places. The subjects were 12 males and 16 females.

The evaluation was performed as follows, and an average value was calculated for each item.

The foreign body feeling was evaluated as 3 points when there was no difference from Comparative Example 2, as 2 points when there was slight concern, and as 1 point when there was pain or concern.

The stickiness was evaluated as 3 points when the stickiness was less than that of Comparative Example 2, as 2 points when the stickiness was not different from that of Reference Example, and as 1 point when the stickiness was more and worse than that of Reference Example.

The gloss was evaluated as 3 points when the gloss was less than that of Comparative Example 2, as 2 points when the gloss was not different from that of Reference Example, and as 1 point when the gloss was more and worse than that of Reference Example.

As for the use feeling, Test Examples 1 and 2 and Comparative Example 1 were ranked in descending order of first, second, and third places (the first place was the best in the use feeling), and were evaluated.

Evaluation results are presented in Table 3. The points and places presented in Table 3 are average values.

**[Table 3]**

| | Test Example 1 | Test Example 2 | Comparative Example 1 | Comparative Example 2 |
|---|---|---|---|---|
| Foreign body feeling | 2.9 | 2.8 | 2.6 | 3.0 |
| Stickiness | 2.2 | 2.3 | 2.2 | 2.0 |
| Gloss | 2.2 | 2.3 | 2.0 | 2.0 |
| Use feeling | 2.4 | 2.1 | 2.6 | - |

An outline of consideration of the sensory test results will be described below. As for the foreign body feeling, less foreign body feeling was felt in Test Examples 1 and 2 than that in Comparative Example 1. As for the stickiness, less stickiness was felt in Test Examples 1 and 2 than that in Comparative Example 2. As for the gloss, gloss was suppressed more in Test Examples 1 and 2 than that in Comparative Examples 1 and 2. As for the use feeling, Test Examples 1 and 2 were ranked at higher places than Comparative Example 1. When Comparative Example 1 was applied to the skin, inorganic fine particles tended to stand out white.

### (Others)

JIS, TAPPI, and other tests and measurement methods described in the above specification are performed at room temperature, particularly 25°C, at atmospheric pressure, particularly 1 atm unless otherwise specified.

### Industrial Applicability

The present invention can be used for an oil-based cosmetic that hardly imparts a foreign body feeling and suppresses stickiness and gloss.

## Claims

1. An oil-based cosmetic comprising:
a fluid oil; and
a cellulose powder,
wherein the cellulose powder contains dried fine fibrous cellulose having an average fiber diameter of 1 to 1000 nm, has an average particle size of 1 to 100 µm, and has a specific surface area of 0.1 to 10 m²/g.

2. The oil-based cosmetic according to claim 1,
wherein the cellulose powder is formed of a composite containing the fine fibrous cellulose and a polyhydric alcohol.

3. The oil-based cosmetic according to claim 1,
further comprising petrolatum.

4. The oil-based cosmetic according to claim 1,
having a B-type viscosity of 100 to 50,000 mPa·s under measurement conditions of 35°C and a rotation speed of 3 rpm.

5. The oil-based cosmetic according to claim 1,
wherein the fluid oil has a melting point of -10°C to 25°C.

6. The oil-based cosmetic according to claim 1,
wherein the fluid oil is liquid paraffin.

7. The oil-based cosmetic according to claim 1,
comprising the cellulose powder in an amount of 0.1 to 20% by mass.

8. The oil-based cosmetic according to claim 1,
wherein the cellulose powder has a moisture percentage of 1 to 20%.

9. The oil-based cosmetic according to claim 1,
wherein the cellulose powder has a loose bulk density of 100 to 1000 mg/cm³.

10. The oil-based cosmetic according to claim 1,
wherein the cellulose powder is white or light yellow.

11. The oil-based cosmetic according to claim 1,
further comprising an additive.

12. The oil-based cosmetic according to claim 2,
wherein the polyhydric alcohol is glycerin.
